(19) Europäisches Patentamt
European Patent Office
Office européen des brevets

(11) EP 2 045 013 A1

(12) EUROPEAN PATENT APPLICATION

(43) Date of publication:
08.04.2009 Bulletin 2009/15

(51) Int Cl.:
B01J 31/12 (2006.01)      B01J 37/02 (2006.01)
B01J 37/18 (2006.01)      C07C 6/10 (2006.01)
C07C 6/12 (2006.01)

(21) Application number: 07253920.8

(22) Date of filing: 03.10.2007

(84) Designated Contracting States:
AT BE BG CH CY CZ DE DK EE ES FI FR GB GR
HU IE IS IT LI LT LU LV MC MT NL PL PT RO SE
SI SK TR
Designated Extension States:
AL BA HR MK RS

(71) Applicants:
• BP OIL INTERNATIONAL LIMITED
Sunbury-on-Thames,
Middlesex TW16 7BP (GB)

• CPE Lyon Formation Continue et Recherche
- CPE Lyon FCR
69100 Villeurbanne (FR)

(72) Inventor: The designation of the inventor has not
yet been filed

(74) Representative: De Kezel, Eric
BP International Limited
Patents & Agreements
Chertsey Road
Sunbury-on-Thames
TW16 7LN (GB)

(54) **Solid metal compound, preparations and uses thereof**

(57) The invention relates to a solid metal compound comprising (i) a solid support comprising aluminium oxide, (ii) at least one first metal compound (C1) selected from metal hydrides, organometallic compounds and organometallic hydrides, and comprising a metal (M1) selected from the lanthanides, the actinides and the metals of Groups 4 to 7 of the Periodic Table of the Elements, and (iii) at least one second metal compound (C2) comprising a metal (M2) selected from the metals of Groups 8 to 10 of said Table. The compounds (C1) and (C2) are preferably supported on, particularly grafted onto the solid support. The invention also relates to processes for preparing the solid metal compound, preferably comprising stage (1) comprising dispersing and preferably grafting (i) an organometallic precursor (Pr1) comprising the metal (M1) and (ii) a precursor (Pr2) comprising the metal (M2) onto the support, so as to produce the solid metal compound, and preferably stage (2) comprising contacting the solid metal compound thus obtained with hydrogen and/or a reducing agent. The invention also relates to the use of the solid metal compound in processes comprising hydrocarbon reactions optionally in the presence of hydrogen, and preferably involving the splitting and recombining of carbon-carbon and/or carbon-hydrogen and/or carbon-metal bonds, so as to produce final hydrocarbons different from the starting ones. The solid metal compound can be used in processes comprising alkane and/or alkene metathesis, non-oxidative methane coupling, alkene oligomerisation, methane-olysis of hydrocarbons, cross-metathesis and hydrogenolysis of hydrocarbons, e.g. saturated hydrocarbons, hydrocarbon polymers/oligomers or waxes, in the presence of hydrogen.

**Description**

The present invention relates to a solid metal compound, its preparation processes and its uses, in particular as a catalyst in metathesis or hydrogenolysis reactions of hydrocarbon compounds.

American Patent US 3,699,035 describes a process for producing gasoline from a hexane-rich hydrocarbon feed. The process comprises averaging hexane with one or more alkanes and catalytically reforming the resulting hydrocarbons. The averaging reaction is carried out at a relatively high temperature, e.g. at about 427°C, using a catalyst mass having hydrocarbon dehydrogenation activity and olefin disproportionation activity. The catalyst mass comprises a component selected from metals of Group 8 to 10 and a component selected from Group 6. More particularly, the preferred catalyst mass consists of platinum on alumina particles mixed with tungsten oxide on silica particles.

International Patent Application WO 98/02244 describes a process for the metathesis of alkanes in which one or more alkanes are reacted on a solid catalyst comprising a metal hydride grafted onto a solid support. The metal of the metal hydride can be chosen from the transition metals of Groups 5 to 6 of the Periodic Table of the Elements, preferably from tantalum, tungsten and chromium. The solid support can be chosen from numerous solid oxides, preferably from silica, alumina, silica-alumina, niobium oxide and zeolites. Amongst the catalysts used for alkane metathesis, it is recommended to select tantalum hydride, tungsten hydride or chromium hydride grafted onto silica or silica-alumina. The examples show the preparations of a tantalum hydride grafted onto a silica and a tungsten hydride grafted onto a silica.

International Patent Application WO 2004/089541 describes a supported metal compound comprising a support based on aluminium oxide onto which is grafted a tungsten hydride. The support can be a mixed aluminium oxide comprising an aluminium oxide combined with at least one other oxide of an element chosen from the metals of Groups 1 to 13 and the Group 14 (with the exception of carbon) of the Periodic Table of the Elements. The other oxide can be an oxide of a metal chosen from the alkaline metals, the alkaline-earth metals, the transition metals and the elements of Groups 13 and 14 (with the exception of carbon) of said Table. More particularly, the other oxide can be an oxide of a transition metal selected from the metals of Groups 3 to 11 of said Table, in particular the elements 21 (i.e. Sc) to 29 (i.e. Cu), 39 (i.e. Y) to 47 (i.e. Ag), 57 (i.e. La) to 79 (i.e. Au) (including the lanthanides) and the actinides. Preferably, the other oxide can be an oxide of a metal chosen from the transition metals of Groups 3 to 7, the lanthanides, the actinides and the elements of Groups 13 and 14 (with the exception of carbon) of said Table. More particularly, the other oxide combined with the aluminium oxide is chosen from oxides of silicon, boron, gallium, germanium, titanium, zirconium, cerium vanadium, niobium, tantalum, chromium, molybdenum and tungsten.

The Periodic Table of the Elements mentioned above and below is that drawn up by the IUPAC in 1991, in which the groups are numbered from 1 to 18. It is found, for instance, in "CRC Handbook of Chemistry and Physics" 76th Edition (1995-1996) by David R. Lide and published by CRC Press, Inc. (USA).

It was found surprisingly that among all the possible compositions of a solid metal compound comprising a tungsten hydride supported onto an aluminium oxide as disclosed in International Patent Application 2004/089541, there was no indication that a specific combination of at least two metals belonging to two different groups of the Periodic Table of the Elements with a solid support based on aluminium oxide, could result in a solid metal compound capable of acting as catalyst and improving catalytic reactions of saturated or unsaturated hydrocarbons. The improvements can be preferably observed in the following reactions: alkane metathesis, alkane hydrogenolysis, alkene (olefin) metathesis, alkene (olefin) oligomerisation, conversion of ethylene into propylene and conversion of isobutene into neohexene. More particularly, these reactions can be performed not only at relatively low temperatures, e.g. lower than 400°C, preferably lower than 350°C or even equal to or lower than 300°C, but also with a substantially enhanced selectivity in the hydrocarbons produced (e.g. alkanes or alkenes), and a catalytic activity which is increased and particularly more stable with time.

The present invention relates to a solid metal compound comprising (i) a solid support comprising aluminium oxide, (ii) at least one first metal compound (C1) selected from metal hydrides, organometallic compounds and organometallic hydrides, and comprising a metal (M1) selected from the lanthanides, the actinides and the metals of Groups 4 to 7 of the Periodic Table of the Elements, and (iii) at least one second metal compound (C2) comprising a metal (M2) selected from the metals of Groups 8 to 10 of said Table.

In the solid metal compound, the support can be a solid support containing aluminium oxide, preferably any solid support where the aluminium oxide is accessible at the surface of said support. The support can be chosen from homogeneous supports comprising aluminium oxide, in particular having a homogeneous composition through the whole mass of the support, i.e. from the centre to the surface of the support. It can also be chosen from heterogeneous supports containing aluminium oxide, in particular comprising aluminium oxide substantially at the surface of the support. In the case of a heterogeneous support, the support can comprise aluminium oxide essentially deposited, supported or grafted on/onto a solid which can itself an organic or preferably an inorganic solid support, more particularly chosen from metals, oxides, sulphides, salts or carbon, preferably from silica and metal oxides.

The support can have a specific surface area (B.E.T.) chosen from 0.1 to 3000 m$^2$/g, preferably from 0.5 to 1000 m$^2$/g, more particularly from 0.5 to 800 m$^2$/g. The specific surface area (B.E.T.) is measured according to the standard ISO 9277 (1995).

The support can be more particularly chosen from aluminium oxide, mixed aluminium oxides and modified aluminium oxides, in particular ones modified by one or more elements of Groups 15 to 17 of the Periodic Table of the Elements.

By aluminium oxide (also simply called alumina) is generally meant an aluminium oxide substantially free from any other oxide (or containing less than 2 wt% of one or more other oxides generally present in the form of impurities). If it contains 2 wt% or more of one or more other oxides, it is generally agreed to regard the oxide as a mixed aluminium oxide, i.e. an aluminium oxide mixed or combined with at least one other oxide.

The support can be an aluminium oxide selected from porous alumina, non-porous alumina and mesoporous alumina.

Porous alumina is often called "activated alumina" or else "transition alumina". It generally corresponds to various partially hydroxylated aluminium oxides. Porous alumina is generally obtained by a so-called "activation" treatment which can comprise a thermal (or dehydration) treatment of a precursor generally selected from hydroxides of aluminium (e.g. tri-hydroxides), hydroxides of aluminium oxide and gelatinous hydroxides of aluminium. The activation treatment makes it possible to remove the water contained in the precursor, and also partially the hydroxyl groups, thus allowing some residual hydroxyl groups and a specific porous structure to remain. The surface of a porous alumina generally comprises a complex mixture of aluminium atoms, oxygen atoms and hydroxyl ions that combine according to specific crystalline forms and which can produce both acid and basic sites. It is thus possible to choose as a solid support a porous alumina selected from γ-alumina (gamma-alumina), η-alumina (eta-alumina), δ-alumina (delta-alumina), θ-alumina (theta-alumina), κ-alumina (kappa-alumina), ρ-alumina (rho-alumina) and χ-alumina (ksi- or chi-alumina), more particularly from γ-alumina and η-alumina. In a porous alumina, the various crystalline forms essentially depend on the choice of the precursor and the conditions of the activation treatment, e.g. the temperature and the pressure. The activation treatment can be carried out under a current of air or another gas, particularly an inert gas, e.g. nitrogen, at a temperature chosen from 100 to 1000°C, preferably from 200 to 1000°C.

The support can be a porous alumina or else more particularly a semi-porous alumina generally obtained by an activation treatment as previously described, in particular at a temperature chosen from 600 to 1000°C. The semi-porous alumina can comprise a mixture of porous alumina with non-porous alumina, in particular a mixture of at least one of the above-mentioned porous aluminas, e.g. selected from γ-alumina, η-alumina, δ-alumina, θ-alumina, χ-alumina, ρ-alumina and κ-alumina, with a non-porous alumina, e.g. α-alumina (alpha-alumina), for instance in a weight ratio of porous to non-porous aluminas chosen from 20:80 to 80:20.

Porous alumina generally is the thermal decomposition product of tri-hydroxides of aluminium, hydroxides of aluminium oxide (or hydrates of aluminium oxide), or gelatinous hydroxides of aluminium (or alumina gels).

Tri-hydroxides of aluminium having the general formula $Al(OH)_3$ or $Al_2O_3,3H_2O$ can exist in various crystalline forms, e.g. gibbsite or hydrargillite ($Al(OH)_3$-α), bayerite ($Al(OH)_3$-β), or nordstrandite. Tri-hydroxides of aluminium can be obtained by precipitation from aluminium salts, preferably in alkaline solutions.

Hydroxides of aluminium oxide having the general formula $AlO(OH)$ or $Al_2O_3,H_2O$ can exist in various crystalline forms, e.g. diaspore ($AlO(OH)$-β) or boehmite ($AlO(OH)$-α). Diaspore can be found in certain types of clay and bauxite, and can be synthesised by a thermal treatment of gibbsite, e.g. at 150°C about, or by a hydrothermal treatment of boehmite, e.g. at 380°C about and under a pressure of 50 MPa. Boehmite can easily be obtained by heating the gelatinous precipitate formed by cold treating solutions of aluminium salts with ammonia. Hydroxides of aluminium oxide can also be obtained by hydrolysis of aluminium alcoholates.

Gelatinous hydroxides of aluminium (or alumina gels) generally are polyhydroxides of aluminium, in particular having the general formula (1):

$$nAl(OH)_3, (n-1)H_2O \qquad (1)$$

wherein n is a number chosen from 1 to 8. Gelatinous hydroxides of aluminium can be obtained by one of the processes chosen from thermal decomposition of an aluminium salt, e.g. aluminium chloride, or from electrolysis of aluminium salts, e.g. a mixture of aluminium sulphate with alkaline sulphate, or from hydrolysis of aluminium alcoholates, e.g. aluminium methylate, or from precipitation from aluminates, e.g. alkaline aluminates or alkaline-earth aluminates, or from precipitation from aluminium salts, e.g. by contacting an aqueous solution of $Al_2(SO_4)_3$ with ammonia, or an aqueous solution of $NaAlO_2$ with an acid, or an aqueous solution of $NaAlO_2$ with an aqueous solution of $Al_2(SO_4)_3$. The precipitates thus obtained can then undergo ageing and drying so as to eliminate water. Gelatinous hydroxides of aluminium generally come in the form of amorphous alumina gels, e.g. in the form of a pseudo-boehmite.

Porous alumina can have a specific surface area (B.E.T.) chosen from 100 to 3000 $m^2/g$, preferably from 100 to 2000 $m^2/g$, particularly from 100 to 1000 $m^2/g$, especially from 200 to 800 $m^2/g$. In addition, porous alumina can exhibit a specific pore volume equal to or less than 1 $cm^3/g$, preferably equal to or less than 0.9 $cm^3/g$, more particularly equal to or less than 0.6 $cm^3/g$.

The support can also be a non-porous alumina, preferably α-alumina (alpha-alumina), generally known under the expression of "calcined alumina" or "flame alumina". In the nature, α-alumina exists under the name of "corundum". It can be generally synthesised by a thermal treatment or a calcinations of a precursor chosen in particular from aluminium salts, hydroxides of aluminium oxide, tri-hydroxides of aluminium and aluminium oxides, e.g. γ-alumina, at a temperature of more than 1000°C, preferably more than 1100°C. It can contains oxide impurities generally selected from $Fe_2O_3$, $SiO_2$, $TiO_2$, CaO, $Na_2O$, $K_2O$, MgO, SrO, BaO and $Li_2O$, generally in proportions of less than 2 wt%, preferably less than 1 wt%. Non-porous alumina, e.g. α-alumina, can have a specific surface area (B.E.T.) chosen from 0.1 to 300 $m^2$/g, preferably from 0.5 to 300 $m^2$/g, more particularly from 0.5 to 250 $m^2$/g.

The support can also be a mesoporous alumina, in particular having a specific surface area (B.E.T.) chosen from 100 to 800 $m^2$/g, preferably from 100 to 600 $m^2$/g. A mesoporous alumina generally can have pores with a width of from 2 nm to 0.05 μm.

The support can also be a mixed aluminium oxide. By mixed aluminium oxide is generally meant an aluminium oxide combined with one or more other oxides, preferably in a proportion of from 2 to less than 80 wt%, more particularly from 2 to less than 50 wt%, in particular from 2 to less than 40 wt%. The other oxide can be an oxide of a metal or an element chosen from the metals and the elements of Groups 1 to 14, with the exception of carbon, of the Periodic Table of the Elements. It can be in particular selected from the alkaline metals, the alkaline-earth metals, the transition metals of Groups 3 to 12, the elements of Group 14 (with the exception of carbon) of the Periodic Table of the Elements, the lanthanides and the actinides. The other oxide combined with the aluminium oxide can be selected from oxides of lithium, sodium, calcium, beryllium, magnesium, zinc, manganese, iron, cobalt, silicon, boron, gallium, germanium, titanium, zirconium, cerium, vanadium, niobium, tantalum, chromium, molybdenum and tungsten. A mixed aluminium oxide can be preferably combined with one or more other oxides selected from oxides of silicon, boron, titanium and niobium, and can be particularly chosen from the following mixed aluminium oxides: $Al_2O_3$/$SiO_2$, $Al_2O_3$/$B_2O_3$, $Al_2O_3$/$SiO_2$/$B_2O_3$, $Al_2O_3$/$TiO_2$ and $Al_2O_3$/$Nb_2O_5$.

A mixed aluminium oxide can be selected from anhydrous aluminates, spinels and alumino-silicates. In particular, the anhydrous aluminates can be chosen from anhydrous alkaline aluminates, e.g. anhydrous lithium aluminate ($LiAlO_2$) or anhydrous sodium aluminate ($Na_2O$, $Al_2O_3$), and from anhydrous alkaline-earth aluminates, e.g. anhydrous tricalcium aluminate (3CaO, $Al_2O_3$) or anhydrous beryllium aluminate (BeO, $Al_2O_3$). The spinels can be particularly chosen from aluminium oxides combined with one or more oxides of divalent metals, e.g. magnesium spinel ($MgAl_2O_4$), calcium spinel ($CaAl_2O_4$), zinc spinel ($ZnAl_2O4$), manganese spinel ($MnAl_2O_4$), iron spinel ($FeAl_2O_4$) or cobalt spinel ($CoAl_2O_4$). The alumino-silicates can be chosen in particular from clays, talcum, micas, feldspar, micro-porous alumino-silicates, in particular molecular sieves, and zeolites.

The support can also be a modified aluminium oxide, in particular one modified by one or more elements of Groups 15 to 17, preferably of Groups 16 and 17 of the Periodic Table of the Elements, more particularly selected from phosphorus, sulphur, fluorine and chlorine. The support can also be selected from alumina superacids, sulphated aluminium oxides, sulphured aluminium oxides, chlorinated aluminium oxides and fluorinated aluminium oxides.

The support can be a homogeneous support comprising aluminium oxide, i.e. having a homogeneous composition in particular through the whole mass of the support. The support can also be a heterogeneous support comprising aluminium oxide, in which support the aluminium oxide, or the mixed aluminium oxide, or the modified aluminium oxide, as previously described, is essentially arranged at the surface of a solid support, the centre of the solid support being essentially composed of an inorganic or organic solid, chosen in particular from metals, oxides, sulphides, salts and carbon, preferably from silica or metal oxides. The heterogeneous support can be prepared by dispersion, precipitation and/or grafting onto the solid of one the above-mentioned precursors based on aluminium oxide. The precursor can be chosen from hydroxides of aluminium, more particularly from tri-hydroxides of aluminium, hydroxides of aluminium oxide and gelatinous hydroxides of aluminium. The gelatinous hydroxides of aluminium as previously described are preferred and are generally known under the expression of "alumina gels" or "amorphous alumina". The preparation of a heterogeneous support can be carried out by employing such a precursor by means of a sol-gel, or with the help of an organometallic compound particularly for the grafting onto the solid.

The support generally comes in the form of particles which can have any shape and any size, preferably a mean size chosen from 10 nm to 10 mm, e.g. from 20 nm to 5 mm. The particles of the support can come as such, or can be shaped so as to have a specific shape, preferably a spherical, spheroidal, hemispherical, hemispheroidal, cylindrical, parallelepipedic or cubic shape, or the shape of rings, pellets, discs or granules.

The solid metal compound of the present invention also comprises at least one first metal compound (C1) selected from metal hydrides, organometallic compounds and organometallic hydrides, said compound (C1) comprising a metal (M1) selected from the lanthanides, the actinides and the metals of Groups 4 to 7, preferably selected from the lanthanides and the metals of Groups 4 to 7, more particularly from the metals of Groups 4 to 7 of the Periodic Table of the Elements.

The first metal compound (C1) may comprise a metal (M1) advantageously selected from lanthanum, cerium, neodymium, samarium, lutetium, titanium, zirconium, hafnium, vanadium, niobium, tantalum, chromium, molybdenum, tungsten and rhenium. Preferably the metal (M1) is selected from zirconium, hafnium, vanadium, niobium, tantalum, chromium, molybdenum, tungsten and rhenium, and more particularly from zirconium, niobium, tantalum, molybdenum, tungsten and rhenium.

The first metal compound (C1) is selected from metal hydrides, organometallic compounds and organometallic hydrides. It can be supported on, or preferably grafted onto the solid support comprising aluminium oxide.

By first metal compound (C1) grafted onto the solid support comprising aluminium oxide is generally meant an atom of the metal (M1) linked to said solid support, preferably by one or more single bonds, particularly to at least one oxygen atom of the aluminium oxide, e.g. according to the general formula (M1-OAl) or (AlO-M1-OAl).

By first metal compound (C1) selected from metal hydrides is generally meant an atom of the metal (M1) linked to one or more hydrogen atoms by a single bond, e.g. according to the general formula (M1-H). The number of hydrogen atoms linked to a metal (M1) atom can depend on the formal oxidation state of said metal (M1) and optionally on the number of single bonds linking said metal (M1) atom to the solid support, particularly to oxygen atom of the aluminium oxide, e.g. according to the general formula (M1-OAl), when the first metal compound (C1) is particularly grafted onto the solid support.

By first metal compound (C1) selected from organometallic compounds is generally meant an atom of the metal (M1) linked to one or more hydrocarbon radicals (R) identical or different, particularly by single or multiple carbon-metal (M1) bonds, e.g. a single bond (M1-R), a double bond (M1=R) or a triple bond (M1≡R). The number of the hydrocarbon radicals (R) linked to a metal (M1) atom can depend on the formal oxidation state of said metal (M1), on the type of the single or multiple carbon-metal (M1) bonds, and optionally on the number of single bonds linking said metal (M1) atom to the solid support, particularly to oxygen atom of the aluminium oxide, e.g. according to the general formula (M1-OAl), when the first metal compound (C1) is particularly grafted onto the solid support.

By first metal compound (C1) selected from organometallic hydrides is generally meant an atom of the metal (M1) simultaneously linked to one or more hydrocarbon radicals (R) identical or different, particularly by single or multiple carbon-metal (M1) bonds, e.g. a single bond (M1-R), a double bond (M1=R) or a triple bond (M1≡R), and to one or more hydrogen atoms by a single bond, e.g. according to the general formula (M1-H). The total number of the hydrocarbon radicals (R) and of the hydrogen atoms linked to a metal (M1) atom can depend on the formal oxidation state of said metal (M1), on the type of the single or multiple carbon-metal (M1) bonds, and optionally on the number of single bonds linking said metal (M1) atom to the solid support, particularly to oxygen atom of the aluminium oxide, e.g. according to the general formula (M1-OAl), when said first metal compound (C1) is particularly grafted onto the solid support.

The first metal compound (C1) is particularly selected from the organometallic compounds and the organometallic hydrides, comprising one or more hydrocarbon radicals (R) identical or different, which can be saturated or unsaturated hydrocarbon radicals, preferably comprising from 1 to 20, particularly from 1 to 14 carbon atoms, and optionally comprising one or more silicon atoms, more particularly in organosilicon (or organosilane) radicals. The hydrocarbon radicals (R) can be chosen from aliphatic or alicyclic radicals, e.g. from alkyl, alkenyl, alkynyl, alkylidene and alkylidyne radicals preferably from $C_1$ to $C_{10}$, particularly linear or branched, or from aryl radicals preferably from $C_6$ to $C_{12}$, or from aralkyl, aralkylidene or aralkylidyne radicals preferably from $C_7$ to $C_{14}$.

The metal (M1) atom of the first metal compound (C1) can be linked to the hydrocarbon radical (R) by one or more single, double or triple carbon-metal (M1) bonds.

A single carbon-metal (M1) bond can be thus involved, in particular a bond of the σ (sigma) type. Thus, the hydrocarbon radical (R) can be an alkyl, alkenyl or alkynyl radical, either linear or branched, or an aryl radical, e.g. a phenyl radical ($C_6H_5$-) or a naphthyl radical ($C_{10}H_9$-), or an aralkyl radical, e.g. a benzyl radical ($C_6H_5$-$CH_2$-) or an ethylbenzyl radical ($C_6H_5$-$CH_2$-$CH_2$-). Generally, by alkyl, alkenyl or alkynyl radical is meant a monovalent aliphatic radical arising from the removal of a hydrogen atom from a carbon atom of the molecule of respectively an alkane, an alkene or an alkyne, or even of an organosilicon (or silane) compound. The hydrocarbon radical (R) may have the general formula (R'-$CH_2$-) wherein R' represents a hydrogen atom or a saturated or unsaturated aliphatic or alicyclic radical, either linear or branched. It can be selected from an alkyl radical e.g. a methyl radical ($CH_3$-), an ethyl radical ($CH_3$-$CH_2$-), a propyl radical ($C_2H_5$-$CH_2$-) or a neopentyl radical (($CH_3$)$_3$C-$CH_2$-), an allyl radical ($CH_2$=CH-$CH_2$-), an alkenyl radical e.g. having the general formula (R'-CH=CH-) wherein R' represents a hydrogen atom or a saturated or unsaturated aliphatic or alicyclic radical either linear or branched, an allenyl radical ($CH_2$=C=CH-), an alkynyl radical e.g. having the general formula (R'-C≡C-) wherein R' represents a hydrogen atom or a saturated or unsaturated aliphatic or alicyclic radical either linear or branched, e.g. an ethynyl radical (CH≡C-), an organosilicon radical e.g. having the general formula (R'$_n$Si-$CH_2$-) wherein R' represents one or more different or identical saturated or unsaturated aliphatic or alicyclic radicals and/or hydrogen atoms and n is a number equal to 1, 2 or 3, e.g. a neosilyl radical (($CH_3$)$_3$Si-$CH_2$-). Generally, by aralkyl radical is meant a monovalent aliphatic radical arising from the removal of a hydrogen atom from a carbon atom of an alkyl radical branched onto an aromatic molecule. Generally, by aryl radical is meant a monovalent aromatic radical arising from the removal of a hydrogen atom from a carbon atom of the ring structure of an aromatic molecule.

A double carbon-metal (M1) bond can be also involved, in particular a bond of the π (pi) type. Thus, the hydrocarbon radical (R) can be an alkylidene radical, either linear or branched, or an aralkylidene radical. Generally, by alkylidene radical is meant a divalent aliphatic radical arising from the removal of two hydrogen atoms from one and the same carbon atom of the molecule of an alkane, an alkene, or an alkyne, or even of an organosilicon (or silane) compound. The hydrocarbon radical (R) can be selected from a methylidene radical ($CH_2=$), an ethylidene radical ($CH_3-CH=$), a propylidene radical ($C_2H_5-CH=$), a neopentylidene radical ($(CH_3)_3C-CH=$) and an allylidene radical ($CH_2=CH-CH=$). It may have the general formula (R'-CH=) wherein R' represents a hydrogen atom or an aliphatic or alicyclic radical, either linear or branched. Generally, by aralkylidene radical is meant a divalent aliphatic radical arising from the removal of two hydrogen atoms from one and the same carbon atom of an alkyl, alkenyl or alkynyl radical branched onto an aromatic molecule.

A triple carbon-metal (M1) bond can be also involved. Thus, the hydrocarbon radical (R) can be an alkylidyne radical, either linear or branched, or an aralkylidyne radical. Generally, by alkylidyne radical is meant a trivalent aliphatic radical arising from the removal of three hydrogen atoms from one and the same carbon atom of the molecule of an alkane, an alkene, or an alkyne, or even of an organosilicon (or silane) compound. The hydrocarbon radical (R) can be selected from an ethylidyne radical ($CH_3-CH\equiv$), a propylidyne radical ($C_2H_5-CH\equiv$), a neopentylidyne radical ($(CH_3)_3C-CH\equiv$) and an allylidyne radical ($CH_2=CH-CH\equiv$). It may have the general formula ($R'-C\equiv$) wherein R' represents a hydrogen atom or an aliphatic or alicyclic radical, either linear or branched. Generally, by aralkylidyne radical is meant a trivalent aliphatic radical arising from the removal of three hydrogen atoms from one and the same carbon atom of an alkyl, alkenyl or alkynyl radical branched onto an aromatic molecule.

More particularly, the hydrocarbon radical (R) can be chosen from the methyl, ethyl, n-propyl, isopropyl, n-butyl, isobutyl, neopentyl, norbornyl, allyl, meta-allyl, neopentylidene, allylidene, neopentylidyne, mesityl, neosilyl radicals.

The first metal compound (C1) may comprise one or more ligands, such as "ancillary" ligands, preferably linked to the metal (M1). The ligand preferably comprises at least one oxygen atom and/or at least one nitrogen atom and/or at least one sulphur atom. It is particular chosen from oxo, alkoxo, aryloxo, aralkyloxo, nitrido, imido, imino, amino and amido ligands. Generally, by oxo, alkoxo, aryloxo, aralkyloxo, nitrido, imido, imino, amino and amido ligands are respectively meant:

- a divalent oxo radical of general formula: $=O$ ,
- a monovalent alkoxo, aryloxo or aralkyloxo radical of general formula: $-OR'$ ,
- a trivalent nitrido radical of general formula: $\equiv N$,
- a divalent imido radical of general formula: $=NR''$ ,
- a monovalent imino radical of general formula: $-N=CH-R''$,
- a monovalent amino or amido radical of general formula: $-NR^1R^2$ ,

general formulae wherein O represents an oxygen atom, N represents a nitrogen atom, R' represents an hydrogen atom or a monovalent hydrocarbon radical selected respectively from alkyl, aryl and aralkyl radicals, R'' represents a hydrogen atom or a monovalent hydrocarbon radical, and $R^1$ and $R^2$, being identical or different, represent a hydrogen atom or a monovalent hydrocarbon radical. More particularly, R', R'', $R^1$ and $R^2$ can be a monovalent radical, either saturated or unsaturated, linear or branched, comprising from 1 to 20, preferably from 1 to 14 carbon atoms, and particularly chosen from alkyl radicals preferably from $C_1$ to $C_{10}$, aryl radicals preferably from $C_6$ to $C_{10}$ or $C_{14}$, and aralkyl radicals preferably from $C_7$ to $C_{14}$. Thus, in the first metal compound (C1), the metal (M1) atom can be bonded to at least one hydrogen atom and/or to at least one hydrocarbon radical (R), and in addition to at least one or more of the above-mentioned ligands, e.g. to the O atom of the oxo radical ($=O$) by a double bond, or to the O atom of the alkoxo, aryloxo or aralkyloxo radical ($-OR'$) by a single bond, or to the N atom of the nitrido radical ($\equiv N$) by a triple bond, or to the N atom of the imido radical ($=NR''$) by a double bond, or to the N atom of the imino radical ($-N=CH-R''$) by a single bond, or else to the N atom of the amino radical ($-NH_2$) or amido radical ($-NR^1R^2$) by a single bond.

The solid metal compound of the present invention also comprises at least one second metal compound (C2) comprising a metal (M2) selected from the metals of Groups 8 to 10 of the Periodic Table of the Elements. The metal (M2) may be selected from iron, ruthenium, osmium, cobalt, rhodium, iridium, nickel, palladium and platinum, and preferably from iron, cobalt and nickel. The second metal compound (C2) preferably comprises a metal (M2) having a formal oxidation state selected from - 4 to + 8, preferably from - 2 to + 6, particularly from 0 to + 4. More particularly, the second metal compound (C2) may comprise a metal (M2) in a reduced state.

The second metal compound (C2) can be supported on, or grafted onto the solid support comprising aluminium oxide and having a type identical to or different from that of the solid support containing the first metal compound (C1). It can be supported on, or grafted onto the same solid support particles as those containing the first metal compound (C1), or different from those containing the first metal compound (C1), the solid support being in particular of a same type or different types.

By second metal compound (C2) grafted onto the solid support comprising aluminium oxide is generally meant an atom of the metal (M2) linked to said solid support, preferably by one or more single bonds, particularly to at least one oxygen atom of the aluminium oxide, e.g. according to the general formula: (M2-OAl) or (AlO-M2-OAl). The atom of the metal (M2) can be linked to an oxygen atom of the aluminium oxide already linked by another oxygen atom to the metal (M1) atom of the first metal compound (C1), e.g. according to the general formula: (M2-OAlO-M1).

More particularly, the second metal compound (C2) can be selected from metal hydrides, organometallic compounds and organometallic hydrides, in particular supported on, preferably grafted onto the solid support.

By second metal compound (C2) selected from metal hydrides is generally meant an atom of the metal (M2) linked to one or more hydrogen atoms by a single bond e.g. according to the general formula (M2-H). The number of hydrogen atoms linked to a metal (M2) atom can depend on the formal oxidation state of said metal (M2) and optionally on the number of single bonds linking said metal (M2) atom to the solid support, particularly to oxygen atom of the aluminium oxide e.g. according to the general formula (M2-OAl), when the second metal compound (C2) is particularly grafted onto the solid support.

By second metal compound (C2) selected from organometallic compounds is generally meant an atom of the metal (M2) linked to one or more hydrocarbon radicals (R) identical or different, particularly by single or multiple carbon-metal (M2) bonds, e.g. a single bond (M2-R), a double bond (M2=R) or a triple bond (M2≡R). The number of the hydrocarbon radicals (R) linked to a metal (M2) atom can depend on the formal oxidation state of said metal (M2), on the type of the single or multiple carbon-metal (M2) bonds, and optionally on the number of single bonds linking said metal (M2) atom to the solid support, particularly to oxygen atom of the aluminium oxide e.g. according to the general formula (M2-OAl), when the second metal compound (C2) is particularly grafted onto the solid support.

By second metal compound (C2) selected from organometallic hydrides is generally meant an atom of the metal (M2) simultaneously linked to one or more hydrocarbon radicals (R) identical or different, particularly by single or multiple carbon-metal (M2) bonds, e.g. a single bond (M2-R), a double bond (M2=R) or a triple bond (M2≡R), and to one or more hydrogen atoms by a single bond e.g. according to the general formula (M2-H). The total number of the hydrocarbon radicals (R) and of the hydrogen atoms linked to a metal (M2) atom can depend on the formal oxidation state of said metal (M2), on the type of the single or multiple carbon-metal (M2) bonds, and optionally on the number of single bonds linking said metal (M2) atom to the solid support, particularly to oxygen atom of the aluminium oxide e.g. according to the general formula (M2-OAl), when the second metal compound (C2) is particularly grafted onto the solid support.

The second metal compound (C2) can be selected from the organometallic compounds and the organometallic hydrides, comprising one or more hydrocarbon radicals (R) identical or different, and having the same definition as for the hydrocarbon radicals (R) as previously defined for the first metal compound (C1).

The second metal compound (C2) may comprise one or more ligands, such as "ancillary" ligands, as previously defined for the first metal compound (C1), and preferably linked to the metal (M2). The ligand of the second metal compound (C2) may be identical or different from that of the first metal compound (C1). In particular, it comprises at least one oxygen atom and/or at least one nitrogen atom and/or at least one sulphur atom. Preferably, the ligand can be selected from sulphate, sulphite, nitrate, acetylacetonate, oxo, alkoxo, aryloxo, aralkyloxo, nitrido, imido, imino, amino and amido ligands. Thus, in the second metal compound (C2), the metal (M2) atom can be linked to at least one or more of the above-mentioned ligands, e.g. to the O atom of the oxo radical (=O) by a double bond, or to the O atom of the alkoxo, aryloxo or aralkyloxo radical (-OR') by a single bond, or to the N atom of the nitrido radical (≡N) by a triple bond, or to the N atom of the imido radical (=NR'') by a double bond, or to the N atom of the imino radical (-N=CH-R'') by a single bond, or else to the N atom of the amino radical (-NH$_2$) or amido radical (-NR$^1$R$^2$) by a single bond.

The second metal compound (C2) can be also selected from the above-mentioned metal hydrides, organometallic compounds and organometallic hydrides, comprising in addition one or more of the above-mentioned ligands, particularly the "ancillary" ligands, and preferably linked to the metal (M2).

The second metal compound (C2) can be a metal selected from the metals (M2) with a formal oxidation state equal to 0, and thus can be present in the form of metal (M2) particles. In this case, the second metal compound (C2) is preferably supported on the solid support.

In the solid metal compound, the weight percentage of the metal (M1) can be selected from 0.1 to 15 %, preferably from 0.5 to 10 %. The weight percentage of the metal (M2) in the solid metal compound can be selected from 0.1 to 15 %, preferably from 0.5 to 10 %. In the solid metal compound, the molar ratio of the metal (M1) to the metal (M2) (i.e. the molar ratio (M1):(M2)) can be selected from 1:100 to 100:1, preferably from 1:16 to 16:1, particularly from 1:10 to 10:1, especially from 1:5 to 5:1. In some cases, it can be selected from 1:100 to 1:1, preferably from 1:50 to 1:1, particularly from 1:30 to 1:1, especially from 1:20 to 1:1.

The present invention also relates to processes for preparing the above-mentioned solid metal compound.

A process for preparing the solid metal compound can comprise generally:

- stage (1) comprising dispersing and preferably grafting (i) an organometallic precursor (Pr1) comprising the metal (M1) linked to at least one hydrocarbon radical (R) and (ii) a precursor (Pr2) comprising the metal (M2) onto the solid support comprising aluminium oxide, either simultaneously or separately, in particular successively in a given order or in a reverse order, so as to produce the solid metal compound, and preferably

- stage (2) comprising contacting the solid metal compound obtained in stage (1) with hydrogen and/or a reducing agent.

Prior to stage (1), the solid support can be preferably subjected to a preliminary stage comprising calcination and/or dehydroxylation of the solid support. Generally, the calcination of the solid support comprises oxidising any carbon present in the support and removing said carbon in the form of carbon dioxide. More particularly, the calcination can be carried out by subjecting the support to an oxidising thermal treatment, preferably in an atmosphere or a current of air, particularly dry air, particularly at a temperature below the fritting temperature of the support, e.g. at a temperature selected from 100 to 1000°C, preferably from 200 to 800°C. The calcination generally can be carried out under an absolute pressure selected in a broad range, e.g. under a sub-atmospheric pressure, preferably selected from $10^{-4}$ Pa to less than $10^2$ kPa, or from $10^{-2}$ Pa to 50 kPa, or the atmospheric pressure, or a supra-atmospheric pressure preferably higher than $10^2$ kPa and lower than 10 MPa. The calcination can be generally carried out for a time sufficient to allow the carbon dioxide to be removed from the support, e.g. a time selected from 10 minutes to 100 hours, preferably from 20 minutes to 48 hours.

Prior to stage (1), the solid support can be also subjected to a preliminary stage comprising dehydroxylation. Generally, the dehydroxylation of the solid support comprises removing any residual water from the support and at least partially hydroxyl groups of support so as to allow a residual quantity of the hydroxyl groups to remain in the support, more particularly at the surface of the support, and/or to produce aluminoxane groups (generally having the formula: Al-O-Al) in the support. More particularly, the dehydroxylation can be carried out by subjecting the support to a thermal treatment generally at a temperature below the fritting temperature of the support, e.g. at a temperature selected from 100 to 1000°C, preferably from 200 to 800°C. The dehydroxylation can be carried out in an inert atmosphere or an inert gas current, preferably in the presence of an inert gas e.g. selected from nitrogen, argon and helium. The dehydroxylation can be also carried out under a sub-atmospheric pressure, e.g. at an absolute pressure selected from $10^{-4}$ Pa to less than $10^2$ kPa, preferably from $10^{-2}$ Pa to 50 kPa. The dehydroxylation is preferably carried out for a time generally sufficient to allow residual quantities of hydroxyl groups to remain in the support and/or aluminoxane groups to be produced in the support, so that said hydroxyl and/or aluminoxane groups are preferably able to react and/or to complex further with the precursors during the preparation of the solid compound. Generally, the dehydroxylation can be carried out for a time selected from 10 minutes to 100 hours, preferably from 20 minutes to 48 hours. Preferably, the preliminary stage comprises first the calcination of the solid support and then the dehydroxylation of the solid support thus calcined.

The organometallic precursor (Pr1) generally comprises the metal (M1) linked to one or more hydrocarbon radicals (R). The metal (M1) atom can be linked to a carbon of the hydrocarbon radical (R) by single, double or triple carbon-metal (M1) bonds. The hydrocarbon radicals (R) can be hydrocarbon radicals, identical or different, saturated or unsaturated, preferably comprising from 1 to 20, particularly from 1 to 14 carbon atoms, and optionally one or more silicon atoms, and can be chosen from the hydrocarbon radicals (R) previously described. They can be particularly selected from aliphatic or alicyclic radicals, e.g. from alkyl, alkenyl, alkynyl, alkylidene or alkylidyne radicals, linear or branched, in particular from $C_1$ to $C_{10}$, or from aryl radicals, in particular from $C_6$ to $C_{12}$, or from aralkyl, aralkylidene or aralkylidyne radicals, in particular from $C_7$ to $C_{14}$. The number of hydrocarbon radicals (R) linked to the metal (M1) atom depends generally on the formal oxidation state of the metal (M1) in the organometallic precursor (Pr1).

The precursor (Pr2) can be selected from organometallic precursors comprising the metal (M2) linked to one or more hydrocarbon radicals (R), identical or different, saturated or unsaturated, preferably comprising from 1 to 20, particularly from 1 to 14 carbon atoms, and optionally comprising one or more silicon atoms, said hydrocarbon radicals (R) being identical to or different from those of the organometallic precursor (Pr1). The hydrocarbon radicals (R) of the precursor (Pr2) generally have same general definitions as those previously given for the hydrocarbon radicals (R) of the organometallic precursor (Pr1).

The precursor (Pr2) can be also selected from metal (M2) salts, preferably from organic metal (M2) salts and inorganic metal (M2) salts, in particular metal (M2) salts which are soluble in water so that the precursor (Pr2) can be preferably used in the form of an aqueous solution of the metal (M2) salts in the process for preparing the solid metal compound. More particularly, the precursor (Pr2) can be a metal (M2) salt selected from metal (M2) sulphates, sulphites, nitrates, nitrites, phosphates, phosphites, borates, fluorides, chlorides, bromides, iodides, alcoholates, carboxylates, oxalates, acetates, glycolates and acetylacetonates.

In stage (1), the precursors (Pr1) and (Pr2) can be dispersed and preferably grafted onto the solid support, either together onto the same solid support particles, or separately onto different solid support particles, the solid support being of the same type or of different types.

In stage (1), dispersing and preferably grafting the precursors (Pr1) and (Pr2) onto the solid support can be carried out by various methods, in particular by a method selected from sublimation, impregnation and dry mixing. Dispersing and preferably grafting the precursors can be monitored during the preparation of the solid metal compound by various methods, e.g. by infrared spectroscopy.

Dispersing and preferably grafting the precursors can be carried out by sublimation. Preferably the sublimation comprises involving the precursors in the solid state generally under standard conditions and heating said precursors in the presence of the solid support at a temperature allowing the precursors to sublime and to migrate in the gaseous state onto the solid support, and preferably to react with said support. The sublimation can be carried out at a temperature selected from - 30 to 200°C, preferably from 20 to 150°C, but lower than the temperature of decomposition of the precursors. The sublimation can be also carried out in an inert atmosphere, preferably in the presence of an inert gas e.g. selected from nitrogen, argon and helium, and particularly under a sub-atmospheric pressure, preferably an absolute pressure selected from $10^{-4}$ Pa to less than $10^2$ kPa, particularly from $10^{-4}$ to 1 Pa. Any excess of the precursor which cannot be dispersed and preferably grafted onto the solid support, can be removed by inverse sublimation.

Dispersing and preferably grafting the precursors can be also carried out by impregnation preferably with the help of a liquid or a solvent. Preferably the impregnation comprises placing the precursors either in suspension in a liquid, or preferably in solution in a solvent, e.g. in an aqueous solution or in an organic solvent either polar or non-polar, e.g. pentane or ethyl ether, and contacting the solid support with said suspension or said solution, preferably with stirring. The impregnation can be carried out at a temperature selected in a broad range, but lower than the temperature of decomposition of the precursors, e.g. a temperature selected from - 80 to 200°C, preferably from 20 to 150°C. The impregnation can be also carried out in an inert atmosphere, preferably in the presence of an inert gas e.g. selected from nitrogen, argon and helium, and under an absolute pressure selected in a broad range, e.g. a sub-atmospheric pressure, the atmospheric pressure or supra-atmospheric pressure. The impregnation generally results in the formation of a suspension of the solid metal compound according to the invention, from which the liquid or the solvent can be removed. Any excess of the precursors which cannot be dispersed and preferably grafted onto the solid support, can be removed by washing with a liquid or a solvent identical to or different from that used for the impregnation.

Dispersing and preferably grafting the precursors can be also carried out by dry mixing, preferably with stirring and particularly with the help of a mechanical mixer. By dry mixing is generally meant a mixing carried out in the absence of liquid or solvent. The dry mixing can comprise involving the precursors in the solid state generally under standard conditions and mixing said precursors with the solid support, in the absence of liquid or solvent, preferably with stirring. The dry mixing can be carried out in an inert atmosphere, preferably in the presence of an inert gas e.g. selected from nitrogen, argon and helium. During or after the dry mixing, a thermal and/or sub-atmospheric treatment can be carried out so as to allow the precursors to migrate onto the solid support and preferably to react with said support. The treatment can be carried out at a temperature selected in a broad range, preferably selected from - 30 to 200°C, particularly from 20 to 150°C. It can be also carried out in an inert atmosphere, preferably in the presence of an inert gas e.g. selected from nitrogen, argon and helium, and preferably under a sub-atmospheric pressure, e.g. under an absolute pressure selected from $10^{-4}$ Pa to less than $10^2$ kPa. Any excess of the precursors which cannot be dispersed or preferably grafted onto the solid support, can be removed by inverse sublimation or by washing with a liquid, preferably an inert organic liquid.

Preferably, stage (1) comprises both (i) dispersing the organometallic precursor (Pr1) and the precursor (Pr2) and (ii) grafting said organometallic precursor (Pr1) and optionally said precursor (Pr2) onto the solid support. More particularly, grafting can be carried out with the solid support previously subjected to the above-described preliminary stage preferably comprising the dehydroxylation of the support. Thus, the dehydroxylation of the support advantageously allows residual quantities of hydroxyl groups to remain in the support and/or aluminoxane groups to be produced in the support, so that said hydroxyl and/or aluminoxane groups can further react and/or complex with the organometallic precursor (Pr1) and optionally the precursor (Pr2), and thus lead to the grafting.

The preparation of the solid metal compound preferably comprises stage (2) wherein the solid metal compound obtained in stage (1) is preferably contacted with hydrogen and/or a reducing agent. The contacting is preferably carried out so as to produce hydrogenolysis and/or reduction of the first metal compound (C1) comprising the metal (M1) and of the second metal compound (C2) comprising the metal (M2), dispersed and preferably grafted onto the solid support. By hydrogenolysis is generally meant a reaction producing splitting of a molecule into two portions and fixing of hydrogen onto the two split portions. In the present case, the hydrogenolysis generally involves a splitting reaction occurring between the metal (M1) and/or (M2) atom and the carbon atom of the hydrocarbon radicals of the first and second metal compounds (C1) and (C2). It generally results from the contacting, the production of the first metal compound (C1) and optionally the second metal compound (C2), respectively comprising either metal (M1) hydrides or organometallic (M1) hydrides, and optionally either (M2) hydrides or organometallic (M2) hydrides, dispersed and preferably grafted onto the solid support. Thus, during the hydrogenolysis, the first metal compound (C1) can be produced for example according to the following equations (2) to (5):

$$M1\text{-}R + H_2 \rightarrow M1\text{-}H + R\text{-}H \qquad (2)$$

$$(R)M1\text{-}R + H_2 \rightarrow (R)M\text{-}1\text{-}H + R\text{-}H \qquad (3)$$

$$Al\text{-}O\text{-}M1\text{-}R + H_2 \rightarrow Al\text{-}O\text{-}M1\text{-}H + R\text{-}H \qquad (4)$$

$$\text{Al-O-(R)M1-R} + H_2 \rightarrow \text{Al-O-(R)M1-H} + \text{R-H} \qquad (5)$$

wherein M1 represents the metal (M1), R represents an hydrocarbon radical as previously described, H represents an hydrogen atom, $H_2$ represents the molecule of hydrogen, Al represents an aluminium atom and O represents an oxygen atom. More particularly, equation (2) represents the hydrogenolysis of an organometallic (M1) compound comprising one hydrocarbon radical (R) leading to the production of a metal (M1) hydride dispersed onto the solid support (not represented in equation (1)). Equation (3) represents the hydrogenolysis of an organometallic (M1) compound comprising two hydrocarbon radicals (R) leading to the production of an organometallic (M1) hydride dispersed onto the solid support (not represented in equation (2)). Equation (4) represents the hydrogenolysis of an oganometallic (M1) compound comprising one hydrocarbon radical (R) and grafted onto the solid support (based on aluminium oxide) leading to the production of a metal (M1) hydride grafted onto said solid support. Equation (5) represents the hydrogenolysis of an organometallic (M1) compound comprising two hydrocarbon radicals (R) and grafted onto the solid support (based on aluminium oxide) leading to the production of an organometallic (M1) hydride grafted onto said solid support.

In a similar way, the second metal compound (C2) can be produced during the hydrogenolysis for example according to the following equations (6) to (9):

$$\text{M2-R} + H_2 \rightarrow \text{M2-H} + \text{R-H} \qquad (6)$$

$$\text{(R)M2-R} + H_2 \rightarrow \text{(R)M2-H} + \text{R-H} \qquad (7)$$

$$\text{Al-O-M2-R} + H_2 \rightarrow \text{Al-O-M2-H} + \text{R-H} \qquad (8)$$

$$\text{Al-O-(R)M2-R} + H_2 \rightarrow \text{Al-O-(R)M2-H} + \text{R-H} \qquad (9)$$

wherein M2 represents the metal (M2) and the other symbols have the same meanings as in the equations (2) to (5). Equation (6) represents the hydrogenolysis of an organometallic (M2) compound comprising one hydrocarbon radical (R) leading to the production of a metal (M2) hydride dispersed onto the solid support (not represented in equation (6)). Equation (7) represents the hydrogenolysis of an organometallic (M2) compound comprising two hydrocarbon radicals (R) leading to the production of an organometallic (M2) hydride dispersed onto the solid support (not represented in equation (7)). Equation (8) represents the hydrogenolysis of an organometallic (M2) compound comprising one hydrocarbon radical (R) and grafted onto the solid support (based on aluminium oxide) leading to the production of a metal (M2) hydride grafted onto said solid support. Equation (9) represents the hydrogenolysis of an organometallic (M2) compound comprising two hydrocarbon radicals (R) and grafted onto the solid support (based on aluminium oxide) leading to the production of an organometallic (M2) hydride grafted onto said solid support.

Generally, it results from stage (2) the production of the first metal compound (C1) and/or the second metal compound (C2) respectively comprising the metals (M1) and (M2) which can be at least partially in a reduced state.

Stage (2) can be carried out in the presence of a hydrogen atmosphere, e.g. under a hydrogen partial pressure selected from 1 kPa to 10 MPa, or a combination of hydrogen with an inert gas, or else in the presence of an inert atmosphere when a reducing agent is used, preferably under an absolute pressure selected from $10^{-2}$ to 10 MPa, in particular at a temperature selected from 20 to 500°C, preferably from 20 to 250°C, and more particularly for a time of contacting selected from 10 minutes to 48 hours, preferably from 20 minutes to 24 hours.

The reducing agent used in stage (2) can be selected from hydrogen and particularly from hydrides, preferably hydrides having a metal or an element selected from Groups 13 and 14 of the Periodic Table of the Elements, e.g. selected from aluminium hydrides, boron hydrides, tin hydrides and silanes.

Various methods can be carried out for preparing the solid metal compound of the invention, particularly when both the precursors (Pr1) and (Pr2) are selected from organometallic precursors comprising one or more hydrocarbon radicals (R) identical or different, saturated or unsaturated, and respectively linked to the metals (M1) and (M2). Thus, for example, the process for preparing the solid metal compound may comprise:

- stage (1) comprising dispersing and preferably grafting, either simultaneously or separately, in particular successively in a given order or in a reverse order, the organometallic precursor (Pr1) and the precursor (Pr2) onto the solid support, said precursor (Pr2) being selected from the organometallic precursors comprising the metal (M2) preferably linked to one or more hydrocarbon radicals (R) identical or different, saturated or unsaturated, so as to produce the solid metal compound preferably comprising metal (M1)/(M2) hydrocarbon compounds supported on, or grafted onto said support, and preferably

- stage (2) comprising contacting the solid metal compound obtained in stage (1) with hydrogen and/or a reducing agent.

The solid metal compound thus obtained after stage (1) may generally comprise organometallic (M1)/(M2) compounds supported on, or grafted onto the solid support. The solid metal compound obtained after stages (1) and (2) may generally comprise metal (M1)/(M2) hydrides and optionally organometallic (M1)/(M2) hydrides supported on, or grafted onto the solid support.

Various methods can be also carried out for preparing the solid metal compound of the invention, particularly when the precursor (Pr1) is selected from the above-mentioned organometallic precursors, preferably comprising one or more hydrocarbon radicals (R) identical or different and linked to the metal (M1), while the precursor (Pr2) is selected from the above-mentioned metal (M2) salts, preferably metal (M2) inorganic salts, in particular water soluble metal (M2) inorganic salts. Thus, the process for preparing the solid metal compound may comprise:

- stage (a) comprising impregnating the solid support with an aqueous solution of the precursor (Pr2) selected from metal (M2) salts,
- stage (b) comprising drying and subjecting to calcination and/or preferably to dehydroxylation the solid product obtained in stage (a),
- stage (c) comprising reducing the metal (M2) by contacting the solid product obtained in stage (b) with hydrogen and/or a reducing agent,
- optionally stage (d) comprising desorbing hydrogen from the solid product obtained in stage (c) when hydrogen is used,
- stage (e) comprising dispersing and preferably grafting the organometallic precursor (Pr1) onto the solid product obtained in stage (c) or (d), so as to produce the solid metal compound, and preferably
- stage (f) comprising contacting the solid metal compound obtained in stage (e) with hydrogen, so as to subject the organometallic (M1) compound dispersed and preferably grafted onto the solid support preferably to hydrogenolysis.

The present invention further relates to the use of the solid metal compound previously described, essentially as a catalyst, in a process involving hydrocarbon reactions, preferably comprising contacting said solid metal compound with one or more starting hydrocarbons optionally in the presence of hydrogen, and involving the splitting and recombining of carbon-carbon and/or carbon-hydrogen and/or carbon-metal bonds, so as to produce final hydrocarbon(s) different from the starting hydrocarbons.

More particularly, the present invention relates to the use of the solid metal compound, essentially as a catalyst, in a process for manufacturing saturated or unsaturated hydrocarbon(s) having a modified carbon skeleton. The process preferably comprises contacting in the presence of said solid metal compound and optionally of hydrogen, at least one saturated or unsaturated aliphatic hydrocarbon with itself, or with at least one other saturated or unsaturated aliphatic hydrocarbon, or else with at least one aromatic hydrocarbon substituted by at least one hydrocarbon radical, or with at least one saturated or unsaturated alicyclic hydrocarbon substituted by at least one hydrocarbon radical, e.g. an alkyl, alkenyl or alkynyl radical. The hydrocarbons used in these processes can be saturated or unsaturated hydrocarbons, preferably selected from alkanes and alkenes (olefins). More particularly, the hydrocarbons involved in these processes can be chosen from:

- linear aliphatic saturated or unsaturated hydrocarbons, in particular from $C_2$ to $C_{30}$, preferably from $C_2$ to $C_{20}$,
- branched aliphatic saturated or unsaturated hydrocarbons, in particular from $C_4$ to $C_{30}$, preferably from $C_4$ to $C_{20}$,
- aromatic hydrocarbons substituted by at least one hydrocarbon radical and particularly selected from $C_7$ to $C_{30}$, preferably from $C_7$ to $C_{20}$, said hydrocarbon radical preferably being linear or branched and selected from $C_1$ to $C_{24}$, preferably from $C_1$ to $C_{14}$,
- alicyclic saturated or unsaturated hydrocarbons substituted by at least one hydrocarbon radical and particularly selected from $C_4$ to $C_{30}$, preferably from $C_4$ to $C_{20}$, said hydrocarbon radical preferably being linear or branched and selected from $C_1$ to $C_{27}$, preferably from $C_1$ to $C_{17}$.

More particularly, the solid metal compound can be used, essentially as a catalyst, in a process comprising alkane metathesis reactions, preferably metathesis reactions of an alkane with itself, i.e. alkane self-metathesis reactions, or metathesis reactions of an alkane with at least one other alkane, i.e. alkane cross-metathesis reactions, the alkane (s) being preferably linear or branched and selected from $C_2$ to $C_{10}$, particularly from ethane, propane, n-butane, isobutane, n-pentane, isopentane, neopentane, n-hexane, isohexane, neohexane, n-heptane, isoheptane, neoheptane, n-octane, isooctane, neooctane, n-nonane, isononane, neononane, n-decane, isodecane and neodecane. The solid metal can be also used in a specific process converting isobutane into 2,3-dimethylbutane. Generally these processes can be carried out at a temperature selected from 20 to 500°C, preferably from 50 to 400°C, particularly from 50 to 350°C, under an absolute pressure particularly selected from 0.1 to 100 MPa, preferably from 0.1 to 50 MPa. The solid metal compound can be particularly used in the processes as described in International Patent Application WO 98/02244.

The solid metal compound can be also used, essentially as a catalyst, in a process comprising alkene (olefin) metathesis reactions, preferably metathesis reactions of an alkene with itself, i.e. alkene self-metathesis reactions, or metathesis reactions of an alkene with at least one other alkene, i.e. alkene cross-metathesis reactions, the alkene(s) being preferably linear or branched, e.g. $\alpha$-olefins (alpha-olefins), and selected from $C_2$ to $C_{10}$, particularly from ethylene, propylene, butene-1, isobutene, trans-butene-2, cis-butene-2, pentene-1, pentene-2, isopentene, hexene-1, hexene-2, hexene-3, isohexene, neohexene, heptene-1, heptene-2, heptene-3, isoheptene, neoheptene, octene-1, octene-2, octene-3, octene-4, isooctene, neooctene, nonene-1, nonene-2, nonene-3, nonene-4, isononene, neononene,decene-1, decene-2, decene-3, decene-4, decene-5, neodecene and isodecene. The solid metal compound can be used in a specific process converting ethylene into propylene as described in French Patent Application FR 2 872 510, or in a process comprising alkene (olefin) metathesis reactions as described in International Patent Application WO 2006/013263. It can be also used in a specific process converting isobutene into neohexene (i.e. 3,3-dimethyl-1-butene).

The solid metal compound can be also used, essentially as a catalyst, in a process comprising alkene (olefin) oligomerisation, preferably ethylene or propylene oligomerisation.

The solid metal compound can be also used, essentially as a catalyst, in a process comprising a non-oxidative coupling reaction of methane, preferably for producing ethane. The process can be carried out at a temperature selected from 20 to 500°C, preferably from 50 to 400°C, particularly from 70 to 350°C, and preferably under an absolute pressure selected from 0.01 to 100 MPa, preferably from 0.1 to 50 MPa. The solid metal compound can be particularly used in the process as described in International Patent Application WO 03/104171.

The solid metal compound can be also used, essentially as a catalyst, in a process comprising a methaneolysis reaction preferably carried out by contacting methane with at least one other aliphatic hydrocarbon, or with at least one aromatic or alicyclic hydrocarbon substituted by at least one hydrocarbon radical. More particularly, the process can comprise contacting, in the presence of the solid metal compound, methane with:

- at least one other aliphatic hydrocarbon chosen from linear aliphatic hydrocarbons particularly from $C_2$ to $C_{30}$, preferably from $C_3$ to $C_{20}$, and from branched aliphatic hydrocarbons particularly from $C_4$ to $C_{30}$, preferably from $C_4$ to $C_{20}$, or
- at least one aromatic hydrocarbon substituted by at least one hydrocarbon radical and particularly selected from $C_7$ to $C_{30}$, preferably from $C_7$ to $C_{20}$, said hydrocarbon radical preferably being linear or branched, saturated or unsaturated, in particular from $C_1$ to $C_{24}$, preferably from $C_1$ to $C_{14}$, e.g. an alkyl, alkenyl or alkynyl radical, or
- at least one alicyclic hydrocarbon substituted by at least one hydrocarbon radical and particularly selected from $C_4$ to $C_{30}$, preferably from $C_4$ to $C_{20}$, said hydrocarbon radical preferably being linear or branched, saturated or unsaturated, in particular from $C_1$ to $C_{27}$, preferably from $C_1$ to $C_{17}$, e.g. an alkyl, alkenyl or alkynyl radical.

The solid metal compound can be also used in a process comprising methane-olysis reactions preferably carried out by contacting methane with natural gas, liquefied petroleum gas or LPG, wet gas or wet natural gas (i.e. a mixture of methane with alkanes of $C_2$ to $C_5$, or $C_3$ and/or $C_4$), natural-gas liquid or NGL, or cuts of light hydrocarbons preferably selected from cuts from $C_1$ to $C_6$, from $C_1$ to $C_5$, from $C_1$ to $C_4$, from $C_1$ to $C_3$, and from $C_1$ to $C_2$.

The solid metal compound can be used in such processes comprising a methane-olysis reaction generally carried out at a temperature selected from 20 to 500°C, preferably from 50 to 400°C, particularly from 70 to 350°C, and preferably at a methane partial pressure selected from 0.1 to 100 MPa, particularly from 0.1 to 50 MPa. The solid metal compound can be particularly used in the methane-olysis processes as described in International Patent Applications WO 01/004077 and WO 03/066552.

The solid metal compound can be also used in a process comprising a cross-metathesis reaction between said solid metal compound and at least one starting hydrocarbon preferably selected from:

- linear or branched aliphatic hydrocarbons, in particular from $C_2$ to $C_{30}$, preferably from $C_2$ to $C_{20}$, or
- alicyclic hydrocarbons substituted by at least one hydrocarbon radical and particularly selected from $C_4$ to $C_{30}$, preferably from $C_4$ to $C_{20}$, said hydrocarbon radical being linear or branched, in particular from $C_1$ to $C_{27}$, preferably from $C_1$ to $C_{17}$, e.g. an alkyl, alkenyl or alkynyl radical.

The solid metal compound can be used in such a cross-metathesis process carried out at a temperature selected from 20 to 500°C, preferably from 50 to 400°C, especially from 70 to 350°C, and preferably under an absolute pressure selected from 0.01 to 50 MPa, preferably from 50 to 20 MPa. It can be particularly used in a cross-metathesis process as described in International Patent Application WO 00/027781.

The solid metal compound can be also used in a process comprising a hydrocarbon hydrogenolysis reaction. Generally, a hydrogenolysis reaction comprises contacting at least one starting hydrocarbon with hydrogen in the presence of the solid metal compound, so as to produce at least one final hydrocarbon having a carbon skeleton modified. The starting hydrocarbon can be selected (i) from saturated hydrocarbons, e.g. the above-mentioned alkanes, (ii) from hydrocarbon polymers or oligomers, e.g. (co)polymers or (co)oligomers of one or more olefinic or vinyl monomers, in particular polyolefins, preferably polyethylene, polypropylene, polybutene-1, polyisobutene, copolymers of ethylene with at least one alpha-olefin from $C_3$ to $C_8$, copolymers of propylene with at least one alpha-olefin from $C_4$ to $C_8$, and copolymers of isobutene with butene-1, or aromatic polyvinyl, preferably polystyrene or polyalphamethylstyrene, or (iii) from hydrocarbon waxes, e.g. Fisher-Tropsh waxes, polyethylene waxes and polypropylene waxes. Generally, such processes can be carried out at a temperature selected from 20 to less than 400°C, preferably from 50 to 300°C, preferably under a hydrogen partial pressure selected from $10^{-3}$ to 20 MPa, preferably from $10^{-2}$ to 10 MPa, in particular for a time selected from 5 minutes to 100 hours, preferably from 10 minutes to 50 hours. The solid metal compound can be particularly used in the process as described in European Patent Application EP 0 840 771.

The following examples illustrate the present invention.

## Example 1: Preparation of a solid metal compound (W,Ni/Al-1) comprising tungsten and nickel compounds and an aluminium oxide support.

In a first stage, there were introduced into a glass reactor with stirring, at 25°C, 1 g of a γ-alumina (gamma-alumina) used as the solid support, having a mean particle size of 40 μm and a specific surface area (B.E.T.) of 200 $m^2$/g, containing 90 wt% of alumina and 9 wt% of water, and sold by Degussa (Germany), and 5 ml of an aqueous solution containing 46 mg of nickel sulphate hexahydrate ($NiSO_4$, $6H_2O$), used as the precursor (Pr2). The mixture thus obtained was maintained under stirring at 25°C for 1 hour. At the end of this time, a water suspension of the alumina support impregnated with nickel sulphate was obtained.

In a second stage, the nickel impregnated alumina support thus obtained was separated from water, and subjected to a treatment comprising drying and calcination, carried out by a dry air current at 500°C, for 3 hours, so as to obtain a nickel product supported on the alumina support.

In a third stage, the nickel product supported on the alumina support thus dried and calcined was subjected to a reduction treatment carried out by subjecting said nickel product to a current of hydrogen in a continuous flow reactor, at 390°C, under an absolute hydrogen pressure of $10^2$ kPa, for 20 hours. At the end of this time, a reduced nickel product supported on the alumina support was thus obtained and then subjected to a hydrogen desorption carried out under vacuum at 500°C for 2 hours.

In a fourth stage, there were introduced into a glass reactor, under an argon atmosphere and at 25°C, 900 mg of the nickel product previously obtained, followed by a n-pentane solution containing 150 mg of tungsten tris(neopentyl) neopentylidyne, used as the organometallic precursor (Pr1) and having the general formula (10):

$$W[-CH_2-C(CH_3)_3]_3 \ [\equiv C-C(CH_3)_3] \qquad (10)$$

The mixture thus obtained was kept at 25°C for 3 hours. At the end of this time, an organometallic tungsten compound supported on and grafted onto the alumina support containing the reduced nickel product was obtained. The excess of the organometallic precursor (Pr1) which had not reacted, was removed by washing with n-pentane at 25°C. The organometallic tungsten compound thus supported on and grafted onto the alumina support containing the reduced nickel product was then dried under vacuum.

In a fifth stage, 600 mg of the organometallic tungsten compound supported on and grafted on the alumina support containing the reduced nickel product, as previously obtained, were isolated and subjected in a glass reactor to a hydrogenolysis treatment by contacting with hydrogen, under an absolute hydrogen pressure of 73 kPa, at 150°C, for 15 hours. At the end of this time, the reactor was cooled to 25°C and there was obtained and isolated under an argon atmosphere a solid metal compound (W,Ni/Al-1) according to the invention, comprising an organometallic tungsten hydride (C1) and a nickel compound (C2) supported on and grafted onto the alumina support, and containing 3.7 wt% of tungsten and 1 wt% of nickel.

## Example 2: Preparation of a solid metal compound (W,Ni/Al-2) comprising tungsten and nickel compounds and an aluminium oxide support.

In a preliminary stage, 1 g of the γ-alumina (gamma-alumina) identical to that used in Example 1, as a support, was subjected to a calcinations treatment carried out by contacting with a current of dry air at 500°C for 15 hours, and then to a dehydroxylation treatment carried out by subjecting the alumina support thus calcined to a vacuum under an absolute pressure of $10^{-2}$ Pa, at 500°C, for 15 hours.

In a first stage, there were introduced into a glass reactor, under an argon atmosphere at 25°C, the alumina support previously obtained and simultaneously:

(i) a n-pentane solution containing 150 mg of tungsten tris(neopentyl) neopentylidyne, used as the organometallic precursor (Pr1) and corresponding to the general formula (11):

$$W[-CH_2-C(CH_3)_3]_3 \; [\equiv C-C(CH_3)_3] \qquad (11)$$

(ii) and a n-pentane solution containing 30 mg of nickel bis(meta-allyl), used as the precursor (Pr2) and corresponding to the general formula (12):

$$Ni \; [\eta^3 \; CH_2-C(CH_3)=CH_2]_2 \qquad (12)$$

The mixture thus obtained was kept at 25°C for 3 hours. At the end of this time, organometallic W/Ni compounds supported on and grafted onto the alumina support were obtained. The excess of the precursors (Pr1) and (Pr2) which had not reacted, was removed from the organometallic W/Ni compounds supported on and grafted onto the alumina support by washing with n-pentane at 25°C. The organometallic W/Ni compounds thus supported and grafted were dried under vacuum and contained 3.6 wt% of tungsten and 0.9 wt% of nickel.

In a second stage, 600 mg of the organometallic W/Ni compounds supported on and grafted onto the alumina support, as previously obtained, were isolated and subjected in a glass reactor to a hydrogenolysis treatment carried out by contacting with hydrogen under an absolute hydrogen pressure of 73 kPa at 150°C for 15 hours. At the end of this time, the reactor was cooled to 25°C, and there was obtained and isolated under an argon atmosphere a solid metal compound (W,Ni/Al-2) according to the invention, comprising an organometallic tungsten hydride (C1) and an organometallic nickel hydride (C2) supported on and grafted onto the alumina support, and containing 3.6 wt% of tungsten and 0.9 wt% of nickel.

### Example 3: Preparation of a solid metal compound (Ta,Co/Al) comprising tantalum and cobalt compounds and an aluminium oxide support.

In a preliminary stage, 1 g of a γ-alumina (gamma-alumina) used as a support, identical to that used in Example 1, was subjected to a calcination treatment carried out by contacting with a current of dry air at 500°C for 15 hours, and then to a dehydroxylation treatment carried out by subjecting the alumina support thus calcined to a vacuum, under an absolute pressure of $10^{-2}$ Pa at 500°C for 15 hours.

In a first stage, there were introduced into a glass reactor, under an argon atmosphere at 25°C, the alumina support previously obtained and simultaneously:

(i) a n-pentane solution containing 145 mg of tantalum tris(neopentyl) neopentylidene, used as the organometallic precursor (Pr1) and corresponding to the general formula (13):

$$Ta \; [-CH_2-C(CH_3)_3]_3 \; [\equiv CH-C(CH_3)_3] \qquad (13)$$

(ii) and a n-pentane solution containing 75 mg of cobalt tetra(norbornyl), used as the precursor (Pr2) and corresponding to the general formula (14):

$$Co \; [-C_7H_{11}]_4 \qquad (14)$$

The mixture thus obtained was kept at 25°C for 3 hours. At the end of this time, organometallic Ta/Co compounds supported on and grafted onto the alumina support was obtained. The excess of the precursors (Pr1) and (Pr2) which had not reacted, was removed from the organometallic Ta/Co compounds supported on and grafted onto the alumina support by washing with n-pentane at 25°C. The organometallic Ta/Co compounds thus supported and grafted were dried under vacuum and contained 3.5 wt% of tantalum and 0.8 wt% of cobalt.

In a second stage, 600 mg of the organometallic Ta/Co compounds supported on and grafted onto the alumina support, as previously obtained, were isolated and subjected in a glass reactor to a hydrogenolysis treatment carried out by contacting with hydrogen under an absolute hydrogen pressure of 73 kPa at 150°C for 15 hours. At the end of this time, the reactor was cooled to 25°C, and there was obtained and isolated under an argon atmosphere a solid metal compound (Ta,Co/Al) according to the invention, comprising an organometallic tantalum hydride (C1) and a cobalt compound (C2) supported on and grafted onto the alumina support, and containing 3.5 wt% of tantalum and 0.8 wt% of cobalt.

### Example 4: Preparation of a solid metal compound (Re,Fe/Al) comprising rhenium and iron compounds and an aluminium oxide support.

The preparation of a solid metal invention according to the invention was performed exactly as in Example 3, apart from using, as the organometallic precursor (Pr1) and the precursor (Pr2), respectively

(i) the rhenium neopentyl,neopentylidene,neopentylidyne of the general formula (15):

$$Re[-CH_2-C(CH_3)_3] [\equiv CH-C(CH_3)_3] [\equiv C-C(CH_3)_3] \qquad (15)$$

instead of the tantalum tris(neopentyl)neopenttylidene, and
(ii) the iron bis(mesityl) of the general formula (16):

$$\{Fe [2,4,6-(CH_3)_3C_6H_2]_2\}_2 \qquad (16)$$

instead of the cobalt tetra(nobornyl).

At the end of the first stage, there were obtained organometallic Re/Fe compounds supported on and grafted onto the alumina support, containing 3.7 wt% of rhenium and 0.95 wt% of iron.

In a second stage, 600 mg of the organometallic Re/Fe compounds supported on and grafted onto the alumina support, as previously obtained, were isolated and subjected to a hydrogenolysis treatment carried out under conditions identical to those of the second stage of Example 3. At the end of the second stage, there was obtained a solid metal compound (Re,Fe/Al) according to the invention, comprising an organometallic rhenium hydride (C1) and an iron compound (C2) supported on and grafted onto the alumina support, and containing 3.7 wt% of rhenium and 0.95 wt% of iron.

### Example 5 (comparative): Preparation of a solid metal compound (W/Al) comprising tungsten compound and an aluminium oxide support.

In a preliminary stage, 530 mg of a $\gamma$-alumina (gamma-alumina) used as a solid support, identical to that used in Example 1, were subjected to a calcination treatment carried out by contacting with a current of dry air at 500°C for 15 hours, and then to a dehydroxylation treatment carried out by subjecting the alumina support thus calcined to a vacuum under an absolute pressure of $10^{-2}$ Pa at 500°C for 15 hours.

In a first stage, there were introduced into a glass reactor with stirring, under an argon atmosphere and at 25°C, the alumina previously treated and a n-pentane solution containing 80 mg of tungsten tris(neopentyl)neopentylidyne, used as a precursor (Pr) and having the above-mentioned general formula (10). The mixture thus obtained was kept at 25°C for 3 hours. At the end of this time, an organometallic tungsten compound supported on and grafted onto the alumina support was obtained. The excess of the precursor (Pr) which had not reacted, was removed from the supported and grafted organometallic tungsten compound by washing with n-pentane at 25°C. The supported and grafted organometallic tungsten compound was then dried under vacuum and contained 3.5 wt% of tungsten

In a second stage, 40 mg of the previously obtained organometallic tungsten compound supported on and grafted onto the alumina support were isolated and subjected in a glass reactor to a hydrogenolysis treatment by contacting with hydrogen under an absolute hydrogen pressure of 73 kPa, at 150°C, for 15 hours. At the end of this time, the reactor was cooled to 25°C, and there was obtained and isolated under an argon atmosphere a solid organometallic tungsten hydride supported on and grafted onto the alumina support (W/Al) containing 3.5 wt% of tungsten.

### Example 6: Process for converting isobutene into neohexene, in the presence of W,Ni/Al).

Into a dynamic reactor having a capacity of 5 ml, heated to 150°C and containing 500 mg of the solid metal compound (W,Ni/Al) prepared in Example 1, isobutene was introduced continuously at a rate of 1.52 moles of isobutene per mole of tungsten of the solid metal compound and per minute, under a total absolute pressure of 0.1 MPa. Under these conditions, isobutene was converted into neohexene (i.e. 3,3-dimethyl-1-butene) and 2,3-dimethyl-1-butene, and after 1000 minutes of reaction, the activity of the solid metal compound (W,Ni/Al) and the neohexene molar selectivity were measured and calculated:

-    the activity of the solid metal catalyst determined by the turn over number (TON) and defined by the following equation (10):

$$TON = \text{(number of moles of isobutene having reacted)} / \text{(number of moles of tungsten)} = 280,$$

, and
- the neohexene molar selectivity defined by the following equation (11):

$$\text{molar selectivity}_{(neohexene)} = 100 \text{ x (number of moles of neohexene produced)} / \text{(total number of moles of all the hydrocarbons produced)} = 40 \%$$

**Example 7 (comparative): Process for converting isobutene into neohexene, in the presence of (W/Al).**

In the present example, the same process was carried out exactly as in Example 6, apart from using the solid metal compound (W/Al) prepared in Example 5 (comparative), instead of the solid metal compound (W,Ni/Al-1) prepared in Example 1.

Under these conditions, isobutene was converted into neohexene and 2,3-dimethyl-1-butene, and after 1000 minutes of reaction, the activity of the solid metal compound (W/Al) and the neohexene molar selectivity were measured and calculated:

- RON = 230, and
- neohexene molar selectivity =10%.

It was observed in comparison to the results of Example 6 that the activity of the solid metal compound (W/Al) was substantially lower than in Example 6 with the solid metal compound (W,Ni/Al-1), while the neohexene molar selectivity was drastically lower, by a factor of four times.

**Example 8: Process for converting ethylene into propylene, in the presence of (W,Ni/Al-1).**

Into a dynamic reactor having a capacity of 5 ml, heated to 150°C and containing 500 mg of the solid metal compound (W,Ni/Al-1) prepared in Example 1, ethylene was continuously introduced at a rate of 1.52 moles of ethylene per mole of tungsten of the solid metal compound and per minute, under a total absolute pressure of 0.1 MPa. Under these conditions, it was observed that ethylene was converted into propylene with a high activity and a very high propylene molar selectivity.

**Example 9: Process comprising propylene metathesis, in the presence of (W,Ni/Al-2).**

Into a dynamic reactor having a capacity of 5 ml, heated to 150°C and containing 500 mg of the solid metal compound (W,Ni/Al-2) prepared in Example 2, propylene was continuously introduced at a rate of 1.52 moles of propylene per mole of tungsten of the solid metal compound and per minute, under a total absolute pressure of 0.1 MPa. Under these conditions, it was observed that propylene was essentially converted into a mixture of ethylene and butenes, with a high activity and very high molar selectivities respectively into ethylene and butanes.

**Example 10: Process comprising propane metathesis, in the presence of (Ta,Co/Al).**

Into a dynamic reactor having a capacity of 5 ml, heated to 150°C and containing 500 mg of the solid metal compound (Ta,Co/Al) prepared in Example 3, propane was continuously introduced at a rate of 1.52 moles of propane par mole of tantalum of the solid metal compound and per minute, under a total absolute pressure of 0.1 MPa. Under these conditions, it was observed that propane was essentially converted into a mixture of ethane and butanes containing very minor amounts of methane, pentanes and hexanes, with a high activity and very high molar selectivities respectively into ethane and butanes.

**Example 12: Process comprising ethane metathesis, in the presence of (Re,Fe/Al).**

Into a dynamic reactor having a capacity of 5 ml, heated to 150°C and containing 500 mg of the solid metal compound (Re,Fe/Al) prepared in Example 4, ethane was continuously introduced at a rate of 1.52 moles of ethane per mole of rhenium of the solid metal compound and per minute, under a total absolute pressure of 0.1 MPa. Under these conditions, it was observed that ethane was essentially converted into a mixture of methane and propane containing very minor amounts of butanes and pentanes, with a high activity and very high molar selectivities respectively into methane and propane.

**Claims**

1. Solid metal compound comprising (i) a solid support comprising aluminium oxide, (ii) at least one first metal compound (C1) selected from metal hydrides, organometallic compounds and organometallic hydrides, and comprising a metal (M1) selected from the lanthanides, the actinides and the metals of Groups 4 to 7 of the Periodic table of the Elements, and (iii) at least one second metal compound (C2) comprising a metal (M2) selected from the metals of Groups 8 to 10 of the Periodic Table of the Elements.

2. Solid metal compound according to claim 1, **characterised in that** the solid support is chosen from aluminium oxide, mixed aluminium oxides and modified aluminium oxides.

3. Solid metal compound according to claim 1 or 2, **characterised in that** the first metal compound (C1) comprises a metal (M1) selected from Groups 4 to 7 of the Periodic Table of the Elements.

4. Solid metal compound according to any one of claims 1 to 3, **characterised in that** the first metal compound (C1) comprises a metal (M1) selected from lanthanum, cerium, neodymium, samarium, lutetium, titanium, zirconium, hafnium, vanadium, niobium, tantalum, chromium, molybdenum, tungsten and rhenium, preferably from zirconium, hafnium, vanadium, niobium, tantalum, chromium, molybdenum, tungsten and rhenium, particularly from zirconium, niobium, tantalum, molybdenum, tungsten and rhenium

5. Solid metal compound according to any one of claims 1 to 4, **characterised in that** the first metal compound (C1) is supported on, preferably grafted onto the solid support.

6. Solid metal compound according to any one of claims 1 to 5, **characterised in that** the second metal compound (C2) comprises a metal (M2) selected from iron, ruthenium, osmium, cobalt, rhodium, iridium, nickel, palladium and platinum, preferably from iron, cobalt and nickel.

7. Solid metal compound according to any one of claims 1 to 6, **characterised in that** the second metal compound (C2) comprises a metal (M2) having a formal oxidation state selected from - 4 to + 8, preferably from - 2 to + 6, particularly from 0 to + 4.

8. Solid metal compound according to any one of claims 1 to 7, **characterised in that** the second metal compound (C2) is selected from metal hydrides, organometallic compounds and organometallic hydrides, in particular supported on, preferably grafted onto the solid support.

9. Solid metal compound according to any one of claims 1 to 8, **characterised in that** the second metal compound (C2) is supported on, or grafted onto the solid support comprising aluminium oxide and being of a type identical to or different from that of the solid support containing the first metal compound (C1).

10. Solid metal compound according to any one of claims 1 to 9, **characterised in that** the second metal compound is supported on, or grafted onto the same solid support particles as those containing the first metal compound (C1), or different from those containing the first metal compound (C1).

11. Solid metal compound according to any one of claims 1 to 10, **characterised in that** the weight percentage of the metal (M1) in said solid metal compound is selected from 0.1 to 15 %, preferably from 0.5 to 10 %.

12. Solid metal compound according to any one of claims 1 to 11, **characterised in that** the weight percentage of the metal (M2) in said solid metal compound is selected from 0.1 to 15 %, preferably from 0.5 to 10 %.

13. Solid metal compound according to any one of claims 1 to 12, **characterised in that** the molar ratio of the metal (M1) to the metal (M2) in said solid metal compound is selected from 1:100 to 100:1, preferably from 1:16 to 16:1, particularly from 1:10 to 10:1.

**14.** Process for preparing the solid metal compound according to any one of claims 1 to 13, **characterised in that** it comprises

- stage (1) comprising dispersing and preferably grafting (i) an organometallic precursor (Pr1) comprising the metal (M1) linked to at one or more hydrocarbon radicals (R) and (ii) a precursor (Pr2) comprising the metal (M2), onto the solid support comprising aluminium oxide, either simultaneously or separately, so as to produce the solid metal compound, and preferably
- stage (2) comprising contacting the solid metal compound obtained in stage (1) with hydrogen and/or a reducing agent.

**15.** Process according to claim 14, **characterised in that** prior to stage (1), the solid support is subjected to a preliminary stage comprising calcination and/or dehydroxylation of the support.

**16.** Process according to claims 14 or 15, **characterised in that** the precursor (Pr2) is selected from organometallic precursors comprising the metal (M2) linked to one or more hydrocarbon radicals (R), identical to or different from those of the organometallic precursor (Pr1).

**17.** Process according to any one of claims 14 to 16, **characterised in that** the organometallic precursor (Pr1) and the precursor (Pr2) are dispersed and preferably grafted onto the solid support, either together onto the same solid support particles, or separately onto different solid support particles, the solid support being of the same type or different types.

**18.** Process according to any one of claims 14 to 17, **characterised in that** dispersing and preferably grafting the organometallic precursor (Pr1) and the precursor (Pr2) onto the solid support are carried out by a method selected from sublimation, impregnation and dry mixing.

**19.** Process according to any one of claims 14 to 18, **characterised in that** it comprises:

- stage (1) comprising dispersing and preferably grafting, either simultaneously or separately, the organometallic precursor (Pr1) and the precursor (Pr2) onto the solid support, said precursor (Pr2) being selected from organometallic precursors comprising the metal (M2) preferably linked to one or more hydrocarbon radicals (R), so as to produce the solid metal compound, and preferably
- stage (2) comprising contacting the solid metal compound obtained in stage (1) with hydrogen and/or a reducing agent.

**20.** Process according to any one of claims 14 to 18, **characterised in that** the precursor (Pr2) is selected from metal (M2) salts, preferably from metal (M2) organic salts and metal (M2) inorganic salts, in particular water soluble metal (M2) salts.

**21.** Process according to claim 20, **characterised in that** it comprises:

- stage (a) comprising impregnating the solid support with an aqueous solution of the precursor (Pr2) comprising the metal (M2) salts,
- stage (b) comprising drying and subjecting to calcination and preferably to dehydroxylation the solid product obtained in stage (a),
- stage (c) comprising reducing the metal (M2) by contacting the solid product obtained in stage (b) with hydrogen and/or a reducing agent,
- optionally stage (d) comprising desorbing hydrogen from the solid product obtained in stage (c) when hydrogen is used,
- stage (e) comprising dispersing and preferably grafting the organometallic precursor (Pr1) onto the solid product obtained in stage (c) or (d), so as to produce the solid metal compound, and preferably
- stage (f) comprising contacting the solid metal compound obtained in stage (e) with hydrogen.

**22.** Use of the solid metal compound according to any one of claims 1 to 13 in a process involving hydrocarbon reactions, preferably comprising contacting said solid metal compound with one or more starting hydrocarbons optionally in the presence of hydrogen, and involving the splitting and recombining of carbon-carbon and/or carbon-hydrogen and/or carbon-metal bonds, so as to produce final hydrocarbon(s) different from the starting hydrocarbons.

**23.** Use of the solid metal compound according to any one of claims 1 to 13 in a process for manufacturing hydrocarbon (s) having a modified carbon skeleton obtained by contacting in the presence of said solid metal compound at least one aliphatic hydrocarbon with itself, or with at least one other aliphatic hydrocarbon, or else with at least one aromatic or alicyclic hydrocarbon substituted by at least one hydrocarbon radical.

**24.** Use of the solid metal compound according to any one of claims 1 to 13 in a process involving alkane metathesis reactions, preferably metathesis reactions of an alkane with itself, or metathesis reactions of an alkane with at least one other alkane, the alkane(s) being preferably linear or branched and selected from $C_2$ to $C_{10}$.

**25.** Use of the solid metal compound according to any one of claims 1 to 13 in a process involving alkene metathesis reactions, preferably metathesis reactions of an alkene with itself, or metathesis reactions of an alkene with at least one other alkene, the alkene(s) being preferably linear or branched, particularly alpha-olefins, and selected from $C_2$ to $C_{10}$.

**26.** Use of the solid metal compound according to any one of claims 1 to 13 in a process converting isobutene into neohexene.

**27.** Use of the solid metal compound according to any one of claims 1 to 13 in a process converting ethylene into propylene.

**28.** Use of the solid metal compound according to any one of claims 1 to 13 in a process comprising alkene oligomerisation, preferably ethylene or propylene oligomerisation.

**29.** Use of the solid metal compound according to any one of claims 1 to 13 in a process comprising a non-oxidative coupling reaction of methane, preferably for producing ethane.

**30.** Use of the solid metal compound according to any one of claims 1 to 13 in a process comprising a methane-olysis reaction preferably carried out by contacting methane with at least one other aliphatic hydrocarbon, or with at least one aromatic or alicyclic hydrocarbon substituted by at least one hydrocarbon radical.

**31.** Use of the solid metal compound according to any one of claims 1 to 13 in a process comprising methane-olysis reactions carried out by contacting methane with natural gas, liquefied petroleum gas, wet gas, wet natural gas, natural-gas liquid or cuts of light hydrocarbon selected from $C_1$ to $C_6$, from $C_1$ to $C_5$, from $C_1$ to $C_4$, from $C_1$ to $C_3$ or from $C_1$ to $C_2$.

**32.** Use of the solid metal compound according to any one of claims 1 to 13 in a process comprising a cross-metathesis reaction between said solid metal compound and at least one starting hydrocarbon.

**33.** Use of the solid metal compound according to any one of claims 1 to in a process comprising a hydrocarbon hydrogenolysis reaction carried out by contacting at least one hydrocarbon with hydrogen.

**34.** Use of the solid metal compound according to claim 33, **characterised in that** the hydrocarbon is selected from saturated hydrocarbon, hydrocarbon polymers or oligomers and waxes.

**European Patent Office**

## EUROPEAN SEARCH REPORT

Application Number

EP 07 25 3920

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2004/089541 A (CPE LYON FORMATION CONTINUE ET [FR]; BASSET JEAN-MARIE [FR]; COPERET C) 21 October 2004 (2004-10-21) * the whole document * ----- | 1-34 | INV. B01J31/12 B01J37/02 B01J37/18 C07C6/10 C07C6/12 |
| A | MOSTAFA TAOUFIK ET AL: "Alumina supported tungsten hydrides, new efficient catalysts for alkane metathesis" TOPICS IN CATALYSIS, KLUWER ACADEMIC PUBLISHERS-PLENUM PUBLISHERS, NE, vol. 40, no. 1-4, 1 November 2006 (2006-11-01), pages 65-70, XP019454946 ISSN: 1572-9028 * the whole document * ----- | 1-34 | |
| A | GOLDMAN A S ET AL: "CATALYTIC ALKANE METHANESIS BY TANDEM ALKANE DEHYDROGENATION - OLEFIN METATHESIS" SCIENCE, AMERICAN ASSOCIATION FOR THE ADVANCEMENT OF SCIENCE,, US, vol. 312, no. 5771, 14 April 2006 (2006-04-14), pages 257-261, XP007900712 ISSN: 0036-8075 * page 258, left-hand column; figure 2 * ----- | 1-34 | |
| A,D | WO 03/104172 A (BP CHEM INT LTD [GB]; COPERET CHRISTOPHE [FR]; DOBSON IAN [GB]; MAUNDE) 18 December 2003 (2003-12-18) * page 9, line 12 - page 10, line 13 * * page 32, line 20 - page 35, line 30 * * page 39, line 32 - page 41, line 17; examples 5,6 * ----- -/-- | 1-31 | TECHNICAL FIELDS SEARCHED (IPC) B01J C07C |

The present search report has been drawn up for all claims

| Place of search | Date of completion of the search | Examiner |
|---|---|---|
| Munich | 3 March 2008 | Bork, Ana-Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

# EUROPEAN SEARCH REPORT

Application Number

EP 07 25 3920

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | VIDAL V ET AL: "METATHESIS OF ALKANES CATALYZED BY SILICA-SUPPORTED TRANSITION METAL HYDRIDES" SCIENCE, AMERICAN ASSOCIATION FOR THE ADVANCEMENT OF SCIENCE,, US, vol. 276, no. 5309, 4 April 1997 (1997-04-04), pages 99-102, XP001205575 ISSN: 0036-8075 * the whole document * | 1-34 | |
| A | MAURY O ET AL: "Catalytic Properties of Silica-Supported Tantalum and Tungsten Hydrides in the Cleavage and Formation of C-C Bonds of Alkanes" 12TH INTERNATIONAL CONGRESS ON CATALYSIS :PROCEEDINGS OF THE ICC 2000. GRANADA, SPAIN, JULY 9 - 14, 2000, STUDIES IN SURFACE SCIENCE AND CATALYSIS, AMSTERDAM : ELSEVIER, NL, vol. PART A VOL. 130, 9 July 2000 (2000-07-09), pages 917-922, XP002258078 ISBN: 0-444-50480-X * the whole document * | 1-34 | |

TECHNICAL FIELDS SEARCHED (IPC)

The present search report has been drawn up for all claims

| Place of search | Date of completion of the search | Examiner |
|---|---|---|
| Munich | 3 March 2008 | Bork, Ana-Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 07 25 3920

This annex lists the patent family members relating to the patent documents cited in the above-mentioned European search report.
The members are as contained in the European Patent Office EDP file on
The European Patent Office is in no way liable for these particulars which are merely given for the purpose of information.

03-03-2008

| Patent document cited in search report | | Publication date | Patent family member(s) | | Publication date |
|---|---|---|---|---|---|
| WO 2004089541 | A | 21-10-2004 | CN | 1795153 A | 28-06-2006 |
| | | | EP | 1603852 A2 | 14-12-2005 |
| | | | FR | 2852866 A1 | 01-10-2004 |
| | | | US | 2007129584 A1 | 07-06-2007 |
| WO 03104172 | A | 18-12-2003 | AU | 2003232358 A1 | 22-12-2003 |
| | | | FR | 2840606 A1 | 12-12-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3699035 A
- WO 9802244 A
- WO 2004089541 A
- FR 2872510
- WO 2006013263 A
- WO 03104171 A
- WO 01004077 A
- WO 03066552 A
- WO 00027781 A
- EP 0840771 A

**Non-patent literature cited in the description**

- **DAVID R. LIDE.** CRC Handbook of Chemistry and Physics. CRC Press, Inc, 1995